# EUROPEAN PATENT APPLICATION

(11) **EP 1 452 176 A1**
(43) Date of publication of application: **01.09.2004**
(21) Application number: 02783727.7
(22) Date of filing: 02.12.2002
(51) Int. Cl.: A61K 31/4245, A61P 3/10, A61P 5/50, A61P 9/12

(54) **INSULIN RESISTANCE IMPROVING AGENTS**

(30) Priority: 03.12.2001 JP 2001369271
(71) Applicant: Takeda Chemical Industries, Ltd., Osaka-shi, Osaka 541-0045 (JP)
(72) Inventor: KAWAHARA, Kiminori, c/o MITSUBISHI PHARMA CORP., Tokyo 103-8405 (JP); NAKAGAWA, Haruto, c/o MITSUBISHI PHARMA CORP., Tokyo 103-8405 (JP); TAMAKAWA, Hiroki, c/o MITSUBISHI PHARMA CORP., Tokyo 103-8405 (JP); KUSUMOTO, Keiji, Mishima-gun, Osaka 618-0011 (JP); YAMAGUCHI, Fuminari, Suita-shi, Osaka 565-0862 (JP); TERASHITA, Zen-ichi, Toyonaka-shi, Osaka 560-0085 (JP)
(74) Representative: Rickard, Timothy Mark Adrian
(86) International application number: PCT/JP2002/012579
(87) International publication number: WO 2003/047573

(57) **Abstract**

The present invention relates to a pharmaceutical agent superior in safety, which shows an excellent effect on insulin resistance improving action and the like for many patients suffering from diseases such as diabetes associated with insulin resistance and the like, and provides an insulin sensitizer, an impaired glucose tolerance improving agent, an agent for the prophylaxis or treatment of diabetes, hyperinsulinemia etc. and the like, which contain 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

## Description

The present invention relates to an insulin resistance (sensitivity) improving agent, an impaired glucose tolerance improving agent, an agent for the prophylaxis or treatment of diabetes (e.g., type II diabetes etc.), hyperinsulinemia and the like, and the like, which comprises a 2-ethoxy-l-[[2'-(5-oxo-2,5-dihydro-1,2,4-oxadiazol-3-yl)biphenyl-4-yl]methyl]-1H-benzimidazole-7-carboxylic acid or a salt thereof.

### Background Art

In recent years, the number of diabetic patients has been increasing, posing a serious social problem. In type II diabetes constituting the majority of diabetes, the insulin action is degraded due to a decreased insulin secretion from β cells of the pancreas, combined with lowered insulin sensitivity (insulin resistance) in insulin target organs such as skeletal muscle, liver, adipose tissue and the like, thereby resulting in hyperglycemia. It is highly likely that the decreased insulin secretion is mainly defined genetically, and the insulin resistance is considered to be largely attributable to obesity caused by environmental factors such as overeating, high-fat foods, lack of exercise and the like, in addition to genetic factors. The co-presence of insulin resistance with obesity invites hyperinsulinemia in compensation. Living organisms deal with insulin resistance due to obesity by oversecretion of insulin. However, sustained insulin resistance weakens β cells of the pancreas and gradually degrades insulin secretory capacity, thus proceeding to diabetic state. When hyperglycemic state persists, glucose itself enhances insulin secretion from β cells of pancreas and peripheral insulin resistance, which results in glucose toxicity. A vicious circle is formed here and the level of disorder increases.

2-Ethoxy-1-[[2'-(5-oxo-2,5-dihydro-1,2,4-oxadiazol-3-yl)biphenyl-4-yl]methyl]-1H-benzimidazole-7-carboxylic acid (hereinafter sometimes to be abbreviated as compound A) is known to have an angiotensin II antagonistic action, and to show a superior treatment effect against cardiovascular diseases such as hypertension, cardiac disease, apoplexy, kidney disease, arteriosclerosis etc., and the like (JP-A-5-271228), but it is not known to afford a superior effect as an insulin sensitizer.

Insulin resistance is also a part of the etiology of hypertension, hyperlipidemia and arteriosclerosis, in addition to obesity and diabetes, where its mechanism is considered to be based on the aforementioned compensated hyperinsulinemia. Moreover, it is known that diabetes, hyperlipidemia, hypertension and obesity easily complicate with each other, and as a common factor, insulin resistance can be mentioned. Accordingly, a pharmaceutical agent that reduces insulin resistance is considered to be extremely useful as a drug for the prophylaxis or treatment of lifestyle-related diseases such as diabetes (e.g., type II diabetes), hypertension, hyperlipidemia and the like. The present invention provides a pharmaceutical agent which is superior in safety and which exerts a superior effect on the action to improve insulin resistance etc., and the like, for a number of patients suffering from diseases associated with insulin resistance such as diabetes.

### Disclosure of the Invention

The present inventors have studied from various aspects and considered for the first time the effect of compound A on insulin resistance based on the glucose clamp technique using spontaneously hypertensive rats. As a result, they have found that compound A unexpectedly has a superior insulin resistance improvement action based on its specific chemical structure, and that compound A is extremely useful as an insulin sensitizer, and further as an impaired glucose tolerance improving agent, an agent for the prophylaxis or treatment of diabetes, hyperinsulinemia and the like, and the like.

Accordingly, the present invention relates to
(1) an insulin sensitizer comprising a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(2) an impaired glucose tolerance improving agent comprising a 2-ethoxy-1-[[2'-(5-oxo-2,5-dihydro-1,2,4-oxadiazal-3-yl)biphenyl-4-yl]methyl]-1H-benzimidazole-7-carboxylic acid or a salt thereof,
(3) an agent for the prophylaxis or treatment of diabetes, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(4) an agent for the prophylaxis or treatment of hyperinsulinemia, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(5) an agent for the prophylaxis or treatment of hypertension associated with insulin resistance, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(6) an agent for the prophylaxis or treatment of hypertension associated with impaired glucose tolerance, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(7) an agent for the prophylaxis or treatment of hypertension associated with diabetes, which comprises a 2-ethoxy-1-[[2'- (5-oxo-2,5-dihydro-1,2,4-oxadiazol-3-yl)biphenyl-4-yl]methyl]-1H-benzimidazole-7-carboxylic acid or a salt thereof,
(8) an agent for the prophylaxis or treatment of hypertension associated with hyperinsulinemia, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(9) an agent for improving insulin resistance occurring in association with hypertension, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(10) an agent for improving impaired glucose tolerance occurring in association with hypertension, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(11) an agent for the prophylaxis or treatment of diabetes occurring in association with hypertension, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(12) an agent for the prophylaxis or treatment of hyperinsulinemia occurring in association with hypertension, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(13) a therapeutic agent of hypertension, which is used for treating hypertension of a hypertensive patient showing a fasting blood glucose level of not less than 126 mg/dl, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof,
(14) a method for treating hypertension, which comprises administering 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a hypertensive patient showing a fasting blood glucose level of not less than 126 mg/dl,
(15) a method for improving insulin resistance, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(16) a method for improving impaired glucose tolerance, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(17) a method for the prophylaxis or treatment of diabetes, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(18) a method for the prophylaxis or treatment of hyperinsulinemia, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(19) a method for the prophylaxis or treatment of hypertension associated with insulin resistance, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(20) a method for the prophylaxis or treatment of hypertension associated with impaired glucose tolerance, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(21) a method for the prophylaxis or treatment of hypertension associated with diabetes, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(22) a method for the prophylaxis or treatment of hypertension associated with hyperinsulinemia, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(23) a method for improving insulin resistance occurring in association with hypertension, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(24) a method for improving impaired glucose tolerance occurring in association with hypertension, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(25) a method for the prophylaxis or treatment of diabetes occurring in association with hypertension, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(26) a method for the prophylaxis or treatment of hyperinsulinemia occurring in association with hypertension, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal,
(27) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an insulin sensitizer,
(28) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an impaired glucose tolerance improving agent,
(29) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of diabetes,
(30) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hyperinsulinemia,
(31) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hypertension associated with insulin resistance,
(32) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hypertension associated with impaired glucose tolerance,
(33) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hypertension associated with diabetes,
(34) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hypertension associated with hyperinsulinemia,
(35) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for improving insulin resistance occurring in association with hypertension,
(36) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for improving impaired glucose tolerance occurring in association with hypertension,
(37) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of diabetes occurring in association with hypertension,
(38) use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hyperinsulinemia occurring in association with hypertension,
(39) a pharmaceutical composition for improving insulin resistance, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and pharmacologically acceptable carrier,
(40) a commercial package comprises a composition of the above-mentioned (39), and a written matter associated therewith, said written matter stating that the composition can or should be used for improving insulin resistance,
(41) a pharmaceutical composition for improving impaired glucose tolerance, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(42) a commercial package comprises a composition of the above-mentioned (41), and a written matter associated therewith, said written matter stating that the composition can or should be used for improving impaired glucose tolerance,
(43) a pharmaceutical composition for the prophylaxis or treatment of diabetes, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(44) a commercial package comprises a composition of the above-mentioned (43), and a written matter associated therewith, said written matter stating that the composition can or should be used for the prophylaxis or treatment of diabetes,
(45) a pharmaceutical composition for the prophylaxis or treatment of hyperinsulinemia, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(46) a commercial package comprises a composition of the above-mentioned (45), and a written matter associated therewith, said written matter stating that the composition can or should be used for the prophylaxis or treatment of hyperinsulinemia,
(47) a pharmaceutical composition for the prophylaxis or treatment of hypertension associated with insulin resistance, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(48) a commercial package comprises a composition of the above-mentioned (47), and a written matter associated therewith, said written matter stating that the composition can or should be used for the prophylaxis or treatment of hypertension associated with insulin resistance,
(49) a pharmaceutical composition for the prophylaxis or treatment of hypertension associated with impaired glucose tolerance, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(50) a commercial package comprises a composition of the above-mentioned (49), and a written matter associated therewith, said written matter stating that the composition can or should be used for the prophylaxis or treatment of hypertension associated with impaired glucose tolerance,
(51) a pharmaceutical composition for the prophylaxis or treatment of hypertension associated with diabetes, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(52) a commercial package comprises a composition of the above-mentioned (51), and a written matter associated therewith, said written matter stating that the composition can or should be used for the prophylaxis or treatment of hypertension associated with diabetes,
(53) a pharmaceutical composition for the prophylaxis or treatment of hypertension associated with hyperinsulinemia, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(54) a commercial package comprises a composition of the above-mentioned (53), and a written matter associated therewith, said written matter stating that the composition can or should be used for the prophylaxis or treatment of hypertension associated with hyperinsulinemia,
(55) a pharmaceutical composition for improving insulin resistance occurring in association with hypertension, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(56) a commercial package comprises a composition of the above-mentioned (55), and a written matter associated therewith, said written matter stating that the composition can or should be used for improving insulin resistance occurring in association with hypertension,
(57) a pharmaceutical composition for improving impaired glucose tolerance occurring in association with hypertension, which comprises 2-ethoxy-1-[[2'-(5-oxo-2,5-dihydro-2,2,4-oxadiazol-3-yl)biphenyl-4-yl]methyl]-1H-benzimidazole-7-carboxylic acid or a salt thereof, and a pharmacologically acceptable carrier,
(58) a commercial package comprises a composition of the above-mentioned (57), and a written matter associated therewith, said written matter stating that the composition can or should be used for improving impaired glucose tolerance occurring in association with hypertension,
(59) a pharmaceutical composition for the prophylaxis or treatment of diabetes occurring in association with hypertension, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(60) a commercial package comprises a composition of the above-mentioned (59), and a written matter associated therewith, said written matter stating that the composition can or should be used for the prophylaxis or treatment of diabetes occurring in association with hypertension,
(61) a pharmaceutical composition for the prophylaxis or treatment of hyperinsulinemia occurring in association with hypertension, which comprises 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, and a pharmacologically acceptable carrier,
(62) a commercial package comprises a composition of the above-mentioned (61), and a written matter associated therewith, said written matter stating that the composition can or should be used for the prophylaxis or treatment of hyperinsulinemia occurring in association with hypertension, and the like.

The 2-ethoxy-1-[[2'-(5-oxo-2,5-dihydro-1,2,4-oxadiazol-3-yl)biphenyl-4-yl]methyl]-1H-benzimidazole-7-carboxylic acid (compound A) used for the insulin sensitizer, impaired glucose tolerance improving agent, an agent for the prophylaxis or treatment of diabetes, hyperinsulinemia and the like, and the like (hereinafter sometimes to be abbreviated as insulin sensitizer etc.) of the present invention is a compound represented by the formula:

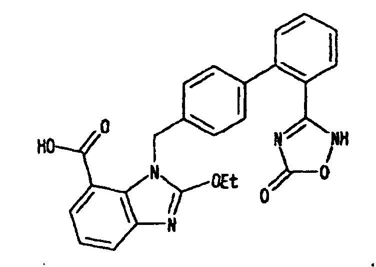

The compound A to be used in the present invention may be the compound per se or a pharmacologically acceptable salt thereof. As such salt, salts with inorganic bases (e.g., alkali metals such as sodium, potassium etc., alkaline earth metals such as calcium, magnesium etc., transition metals such as zinc, iron, copper etc., and the like), salts with organic bases (e.g., organic amines such as trimethylamine, triethylamine, pyridine, picoline, ethanolamine, diethanolamine, triethanolamine, dicyclohexylamine, N,N'-dibenzylethylenediamine etc., salts with basic amino acids such as arginine, lysine and ornithine etc. etc., and the like) and the like, salts with inorganic acids or organic acids (e.g., hydrochloric acid, nitric acid, sulfuric acid, phosphoric acid, carbonic acid, bicarbonic acid, formic acid, acetic acid, propionic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid etc.), and salts with acidic amino acids such as aspartic acid and glutamic acid etc. can be mentioned.

The compound A and a pharmacologically acceptable salt thereof show low toxicity and can be used as a safe insulin sensitizer etc. to mammals (e.g., human, mouse, rat, rabbit, dog, cat, bovine, swine, monkey and the like) in the form of the compound as it is or a pharmaceutical composition after admixing with a pharmacologically acceptable carrier according to a method known *per se.*

As used herein, as the pharmacologically acceptable carrier, various organic or inorganic carrier substances conventionally used as materials for preparations can be used. For example, excipient, lubricant, binder and disintegrant for solid preparations; solvent, dissolution aids, suspending agent, isotonizing agent, buffer and soothing agent for liquid preparations; and the like can be mentioned. Where necessary, additives for preparation, such as preservative, antioxidant, coloring agent, sweetening agent and the like, can be also used.

Preferable examples of excipient include lactose, sucrose, D-mannitol, D-sorbitol, starch, pregelatinized starch, dextrin, crystalline cellulose, low-substituted hydroxypropyl cellulose, carboxymethyl cellulose sodium, gum arabic, dextrin, pullulan, light silicic anhydride, synthetic aluminum silicate, magnesium aluminometasilicate and the like.

Preferable examples of lubricant include magnesium stearate, calcium stearate, talc, colloidal silica and the like.

Preferable examples of binder include pregelatinized starch, sucrose, gelatin, gum arabic, methyl cellulose, carboxymethyl cellulose, carboxymethyl cellulose sodium, crystalline cellulose, sucrose, D-mannitol, trehalose, dextrin, pullulan, hydroxypropyl cellulose, hydroxypropylmethyl cellulose, polyvinylpyrrolidone and the like.

Preferable examples of disintegrant include lactose, sucrose, starch, carboxymethyl cellulose, carboxymethyl cellulose calcium, croscarmellose sodium, carboxymethyl starch sodium, light silicic anhydride, low-substituted hydroxypropyl cellulose and the like.

Preferable examples of solvent include water for injection, physiological brine, Ringer's solution, alcohol, propylene glycol, polyethylene glycol, sesame oil, corn oil, olive oil, cottonseed oil and the like.

Preferable examples of dissolution aids include polyethylene glycol, propylene glycol, D-mannitol, trehalose, benzyl benzoate, ethanol, Tris aminomethane, cholesterol, triethanolamine, sodium carbonate, sodium citrate, sodium salicylate, sodium acetate and the like.

Preferable examples of suspending agent include surfactants such as stearyltriethanolamine, sodium lauryl sulfate, lauryl aminopropionate, lecithin, benzalkonium chloride, benzethonium chloride, monostearic glycerol etc.; hydrophilic polymers such as polyvinyl alcohol, polyvinylpyrrolidone, carboxymethylcellulose sodium, methylcellulose, hydroxymethylcellulose, hydroxyethylcellulose, hydroxypropylcellulose etc.; polysorbates, polyoxyethylene hydrogenated castor oil and the like.

Preferable examples of isotonizing agent include sodium chloride, glycerin, D-mannitol, D-sorbitol, glucose and the like.

Preferable examples of buffer include buffers such as phosphate, acetate, carbonate, citrate etc., and the like.

Preferable examples of soothing agent include benzyl alcohol and the like.

Preferable examples of preservative include p-oxybenzoic acid esters, chlorobutanol, benzyl alcohol, phenethyl alcohol, dehydroacetic acid, sorbic acid and the like.

Preferable examples of antioxidant include sulfite, ascorbic acid salt and the like.

Preferable examples of coloring agent include water-soluble edible tar dyes (e.g., food colors such as Food Red Nos. 2 and 3, Food Yellow Nos. 4 and 5, Food Blue Nos. 1 and 2 etc.), water-insoluble Lake dyes (e.g., aluminum salts of the aforementioned water-soluble edible tar dyes etc.), natural colors (e.g., β-carotin, chlorophyll, iron oxide red etc.) and the like.

Preferable examples of sweetening agent include saccharin sodium, dipotassium glycyrrhizinate, aspartame, stevia and the like.

The dosage form of the pharmaceutical composition includes, for example, oral agents such as tablet, capsule (including soft capsule and microcapsule), granule, powder, syrup, emulsion, suspension, sustained-release preparation and the like; and parenteral agents such as injections (e.g., subcutaneous injection, intravenous injection, intramuscular injection, intraperitoneal injection, intravitreous injection etc.), drop, external agents (e.g., nasal administration preparation, transdermal preparation, ointment etc.), suppositories (e.g., rectal suppository, vaginal suppositories etc.), pellet, drop, and the like, which can be each safely administered orally or parenterally.

The pharmaceutical composition can be prepared by conventional methods in the field of pharmaceutical manufacturing technical field, such as methods described in the Japanese Pharmacopoeia, and the like. Specific production methods for such preparations are hereinafter described in detail.

For example, an oral agent is produced by adding, for example, excipients (e.g., lactose, sucrose, starch, D-mannitol etc.), disintegrants (e.g., carboxymethyl cellulose calcium etc.), binders (e.g., pregelatinized starch, gum arabic, carboxymethyl cellulose, hydroxypropyl cellulose, polyvinylpyrrolidone etc.), lubricants (e.g., talc, magnesium stearate, polyethylene glycol 6000 etc.) and the like, to the active ingredient, compression-shaping, and, where necessary, applying a coating by a method known per se using coating base for the purpose of achieving taste masking, enteric dissolution or sustainability.

As the coating base, for example, a sugar coating base, a water-soluble film coating base, an enteric film coating base, a sustained-release film coating base and the like can be mentioned.

As the sugar coating base, sucrose is used, and one or more kinds selected from talc, precipitated calcium carbonate, gelatin, gum arabic, pullulan, carnauba wax and the like may be further used in combination.

As the water-soluble film coating base, for example, cellulose polymers such as hydroxypropyl cellulose, hydroxypropylmethyl cellulose, hydroxyethyl cellulose, methylhydroxyethyl cellulose etc.; synthesis polymers such as polyvinylacetal diethylaminoacetate, aminoalkylmethacrylate copolymer E [Eudragit E (trademark), Rohm Pharma], polyvinylpyrrolidone etc.; polysaccharides such as pullulan etc.; and the like can be mentioned.

As the enteric film coating base, for example, cellulose polymers such as hydroxypropylmethylcellulose phthalate, hydroxypropylmethylcellulose acetate succinate, carboxymethylethylcellulose, cellulose acetate phthalate etc.; acrylic acid polymers such as methacrylic acid copolymer L [Eudragit L (trademark), Rohm Pharma], methacrylic acid copolymer LD [Eudragit L-30D55 (trademark), Rohm Pharma], methacrylic acid copolymer S [Eudragit S (trademark), Rohm Pharma] etc.; naturally occurring substances such as shellac etc.; and the like can be mentioned.

As the sustained-release film coating base, for example, cellulose polymers such as ethylcellulose etc.; acrylic acid polymers such as aminoalkylmethacrylate copolymer RS [Eudragit RS (trademark), Rohm Pharma], ethyl acrylate-methyl methacrylate copolymer suspension [Eudragit NE (trademark), Rohm Pharma] etc.; and the like can be mentioned.

The aforementioned coating bases may be used after mixing with two or more kinds thereof at appropriately ratios. For coating, for example, shading agents such as titanium oxide, red ferric oxide and the like may be used.

Injections are produced by dissolving, suspending or emulsifying the active ingredient in, for example, aqueous solvents (e.g., distilled water, physiological brine, Ringer's solution etc.), oily solvents (e.g., vegetable oils such as olive oil, sesame oil, cottonseed oil, corn oil etc., propylene glycol etc.) and the like, together with dispersing agents (e.g., polysorbate80, polyoxyethylene hydrogenated castor oil 60, polyethylene glycol, carboxymethylcellulose, sodium alginate etc.), preservatives (e.g., methylparaben, propylparaben, benzyl alcohol, chlorobutanol, phenol etc.), isotonizing agents (e.g., sodium chloride, glycerin, D-mannitol, D-sorbitol, glucose etc.) and the like. Where desired, additives such as dissolution aids (e.g., sodium salicylate, sodium acetate etc.), stabilizers (e.g., human serum albumin etc.), soothing agents (e.g., benzyl alcohol etc.) and the like may be used.

The content of compound A or a salt thereof in a pharmaceutical composition is generally about 0.01 - about 99.9 wt%, preferably about 0.1 - about 50 wt%, relative to the entire preparation.

While the dose of compound A or a pharmacologically acceptable salt thereof varies depending on the subject of administration, administration route, target disease, clinical condition and the like, in the case of, for example, oral administration to a mammal, particularly an adult showing insulin resistant (body weight 50 kg), it is generally about 0.001-500 mg, preferably 0.1-50 mg, more preferably 5-50 mg, per dose of the active ingredient (compound A or a pharmacologically acceptable salt thereof), which is desirably administered once to 3 times a day.

In addition, the present invention encompasses a commercial package comprising the above-mentioned pharmaceutical composition, and a written matter relating to the above-mentioned composition, which describes the object of use, method of use and/or dose etc. of the above-mentioned composition.

The compound A normalizes the intracellular insulin signal transduction mechanism, which mainly causes insulin resistance, thereby reducing insulin resistance and enhancing insulin action, and has a glucose tolerance improvement action. Therefore, it can be used for mammals (e.g., human, mouse, rat, rabbit, dog, cat, bovine, horse, swine, monkey etc.) as an improving agent or an agent for the prophylaxis and/or treatment of the diseases in which insulin is involved. As such diseases, for example, insulin resistance, impaired glucose tolerance; diabetes such as noninsulin dependent diabetes, type II diabetes, type II diabetes associated with insulin resistance, type II diabetes associated with impaired glucose tolerance etc.; various complications such as hyperinsulinemia, hypertension associated with insulin resistance, hypertension associated with impaired glucose tolerance, hypertension associated with diabetes (e.g., type II diabetes etc.), hypertension associated with hyperinsulinemia, insulin resistance occurring in association with hypertension, impaired glucose tolerance occurring in association with hypertension, diabetes occurring in association with hypertension, hyperinsulinemia occurring in association with hypertension, diabetic complications [e.g., microangiopathy, diabetic neuropathy, diabetic nephropathy, diabetic retinopathy, diabetic cataract, large vessel disease, osteopenia, diabetic hyperosmolar coma, infectious diseases (e.g., respiratory infectious disease, urinary tract infectious disease, digestive infectious disease, infectious disease of dermal soft tissue, infectious disease of inferior limb etc.), diabetic gangrene, dry mouth, lowered sense of hearing, diabetic cerebrovascular disorder, diabetic peripheric hematogenous disorder, diabetic hypertension and the like], diabetic cachexia, diabetic nephropathy and the like; and the like can be mentioned.

As a target disease of compound A or a salt thereof as a physiologically active compound includes, for example, diseases developed or whose onset is promoted by contraction and growth of blood vessels or organ disorders that express via angiotensin II receptor, by the presence of angiotensin II, or by the factors induced by the presence of angiotensin II and the like can be mentioned.

As such diseases, for example, hypertension, blood pressure circadian rhythm abnormality, heart diseases (e.g., cardiac hypertrophy, acute heart failure and chronic heart failure including congestive heart failure, cardiac myopathy, angina pectoris, myocarditis, arrhythmia, tachycardia, cardiac infraction etc.), cerebrovascular disorders (e.g., asymptomatic cerebrovascular disorder, transient cerebral ischemia, apoplexy, cerebrovascular dementia, hypertensive encephalopathy, cerebral infarction etc.), cerebral edema, cerebral circulatory disorder, recurrence and sequela of cerebrovascular disorders (e.g., neurotic symptom, psychic symptom, subjective symptom, disorder in daily living activities etc.), ischemic peripheral circulation disorder, myocardial ischemia, venous insufficiency, progression of cardiac insufficiency after cardiac infarction, renal diseases (e.g., nephritis, glomerulonephritis, glomerulosclerosis, renal failure, thrombotic vasculopathy, complication of dialysis, organ dysfunction including nephropathy by radiation damage etc.), arteriosclerosis including atherosclerosis (e.g., aneurysm, coronary arteriosclerosis, cerebral arteriosclerosis, peripheral arteriosclerosis etc.), vascular hypertrophy, vascular hypertrophy or obliteration and organ disorders after intervention (e.g., percutaneous transluminal coronary angioplasty, stenting, coronary angioscopy, intravascular ultrasound, dounce thrombolytic therapy etc.), vascular reobliteration and restenosis after bypass, polycythemia, hypertension, organ disorder and vascular hypertrophy after transplantation, rejection after transplantation, ocular diseases (e.g., glaucoma, ocular hypertension etc.), thrombosis, multiple organ disorder, endothelial dysfunction, hypertensive tinnitus, other cardiovascular diseases (e.g., deep vein thrombosis, obstructive peripheral circulatory disorder, arteriosclerosis obliterans, obstructive thromboangiitis, ischemic cerebral circulatory disorder, Raynaud's disease, Berger disease etc.), metabolic and/or nutritional disorders (e.g., obesity, hyperlipidemia, hypercholesterolemia, hyperuricacidemia, hyperkalemia, hypernatremia etc.), nerve degeneration diseases (e.g., Alzheimer's disease, Parkinson's syndrome, amyotrophic lateral sclerosis, AIDS encephalopathy etc.), central nervous system disorders (e.g., cerebral hemorrhage, cerebral infarction, their sequela and complication, head injury, spinal injury, cerebral edema, sensory malfunction, sensory functional disorder, autonomic nervous system disorder, autonomic nervous system malfunction, multiple sclerosis etc.), dementia, defects of memory, disorder of consciousness, amnesia, anxiety symptom, catatonic symptom, discomfort mental state, psychopathies (e.g., depression, epilepsy, alcoholism etc.), inflammatory diseases (e.g., arthritis such as rheumatoid arthritis, osteoarthritis, rheumatoid myelitis, periostitis etc.; inflammation after operation and injury; remission of swelling; pharyngitis; cystitis; pneumonia; atopic dermatitis; inflammatory intestinal diseases such as Crohn's disease, ulcerative colitis etc.; meningitis; inflammatory ocular disease; inflammatory pulmonary disease such as pneumonia, pulmonary silicosis, pulmonary sarcoidosis, pulmonary tuberculosis etc.), allergic diseases (e.g., allergic rhinitis, conjunctivitis, gastrointestinal allergy, pollinosis, anaphylaxis etc.), chronic obstructive pulmonary disease, interstitial pneumonia, pneumocytis carinni pneumonia, collagen diseases (e.g., systemic lupus erythematodes, scleroderma, polyarteritis etc.), hepatic diseases (e.g., hepatitis including chronic hepatitis, hepatic cirrhosis etc.), portal hypertension, digestive system disorders (e.g., gastritis, gastric ulcer, gastric cancer, gastric disorder after operation, dyspepsia, esophageal ulcer, pancreatitis, colon polyp, cholelithiasis, hemorrhoidal disease, varices ruptures of esophagus and stomach etc.), blood and/or myelopoietic diseases (e.g., erythrocytosis, vascular purpura, autoimmune hemolytic anemia, disseminated intravascular coagulation syndrome, multiple myelopathy etc.), bone diseases (e.g., fracture, refracture, osteoporosis, osteomalacia, bone Paget's disease, sclerosing myelitis, rheumatoid arthritis, osteoarthritis of the knee and joint tissue dysfunction owing to similar diseases and disorder etc.), solid tumor, tumors (e.g., malignant melanoma, malignant lymphoma, cancer of digestive organs (e.g., stomach, intestine etc.) etc.), cancer and cachexia following cancer, metastasis cancer, endocrinopathy (e.g., Addison's disease, Cushing's syndrome, pheochromocytoma, primary aldosteronism etc.), Creutzfeldt-Jakob disease, urinary organ and/or male genital diseases (e.g., cystitis, prostatic hypertrophy, prostatic cancer, sex infectious disease etc.), female disorders (e.g., climacteric disorder, gestosis, endometriosis, hysteromyoma, ovarian disease, breast disease, sex infectious disease etc.), disease relating to environment and occupational factors (e.g., radiation hazard, hazard by ultraviolet, infrared, or laser beam, altitude sickness etc.), respiratory diseases (e.g., cold syndrome, pneumonia, asthma, pulmonary hypertension, pulmonary thrombosis and pulmonary embolism etc.), infectious diseases (e.g., viral infectious diseases with cytomegalovirus, influenza virus, herpes virus etc., rickettsiosis, bacterial infectious disease etc.), toxemias (e.g., sepsis, septic shock, endotoxin shock, Gram-negative sepsis, toxic shock syndrome etc.), otorhinolaryngological diseases (e.g., Meniere's syndrome, tinnitus, dysgeusia, vertigo, disequilibrium, dysphagia etc.), skin diseases (e.g., keloid, hemangioma, psoriasis etc.), intradialytic hypotension, myasthenia gravis, systemic diseases such as chronic fatigue syndrome and the like can be mentioned.

In addition, long-term suppression of action of angiotensin II results in the improvement or suppression of promotion of disorder or abnormality in the biofunction and physiological action, that causes adult disorders and various diseases linked with aging and the like, which in turn leads to the primary and secondary prophylaxis of diseases or clinical conditions caused thereby or suppression of the progression thereof. As the disorder or abnormality in the biofunction and physiological action, for example, disorder or abnormality in automatic controlling capability of cerebral circulation and/or renal circulation, disorder of circulation (e.g., peripheral, cerebral, microcirculation etc.), disorder of blood-brain-barrier, salt susceptibility, abnormal state of coagulation and fibrinolysis system, abnormal state of blood and blood cell components (e.g., accentuation of platelet aggregation activity, erythrocyte deformability, accentuation of leukocyte adhesiveness, rise of blood viscosity etc.), production and function accentuation of growth factor and cytokines (e.g., PDGF, VEGF, FGF, interleukin, TNF-α, MCP-1 etc.), accentuation of proliferation and infiltration of inflammatory cells, accentuation of production of free radical, liposteatosis accentuation, endothelial function disorder, endothrial, cell and organ dysfunction, edema, cell morphogenesis change of smooth muscle etc. (morphogenesis to proliferation type etc.), production and function accentuation of vasoactive substance and thrombosis inducers (e.g., endothelin, thromboxane A₂ etc.), abnormal constriction of blood vessel etc., metabolic disorder (e.g., serum lipid abnormalities, dysglycemia etc.), abnormal growth of cell etc., angiogenesis (including abnormal vasculogenesis during abnormal capillary reticular formation in adventitial coat of arteriosclerosis) and the like can be mentioned. Of these, the present invention can be used as an agent for the primary and secondary prophylaxis or treatment of organ disorders associated with various diseases (e.g., cerebrovascular disorder and organ disorder associated therewith, organ disorder associated with cardiovascular disease, organ disorder associated with diabetes, organ disorder after intervention etc.). Therefore, the insulin sensitizer etc. of the present invention can be advantageously used when the patients with insulin resistance, impaired glucose tolerance, diabetes or hyperinsulinemia have concurrently developed the above-mentioned diseases.

The new criteria were reported about diabetic criteria in 1999 by the Japan Diabetes Society.

According to this report, diabetes is a condition wherein the fasting blood glucose level (glucose concentration of venous plasma) is not less than 126 mg/dl, the 2-hour value (glucose concentration of venous plasma) of the 75 g oral glucose tolerance test (75 g OGTT) is not less than 200 mg/dl, or the casual blood glucose level (glucose concentration of venous plasma) is not less than 200 mg/dl. In addition, a condition which does not fall under the above-mentioned diabetes, and which is not a "condition where the fasting blood glucose level (glucose concentration of venous plasma) is less than 110 mg/dl or the 2-hour value (glucose concentration of venous plasma) of the 75 g oral glucose tolerance test (75 g OGTT) is less than 140 mg/dl" (normal type), is called a "borderline type".

In addition, regarding diagnostic criteria for diabetes, new diagnostic criteria were reported by ADA (The American Diabetes Association) in 1997 and by WHO in 1998.

According to these reports, diabetes is a condition where the fasting blood glucose level (glucose concentration in venous plasma) is not less than 126 mg/dl, and the 2-hour value (glucose concentration in venous plasma) of the 75 g oral glucose tolerance test is not less than 200 mg/dl.

In addition, according to the above reports, impaired glucose tolerance is a condition where the fasting blood glucose level (glucose concentration in venous plasma) is less than 126 mg/dl, and the 2-hour value (glucose concentration in venous plasma) of the 75 g oral glucose tolerance test is not less than 140 mg/dl and less than 200 mg/dl. Furthermore, according to the ADA report, a condition where the fasting blood glucose level (glucose concentration in venous plasma) is not less than 110 mg/dl and less than 126 mg/dl, is called IFG (Impaired Fasting Glucose). On the other hand, according to the WHO report, of the conditions of IFG (impaired fasting glucose), a condition where the 2-hour value (glucose concentration in venous plasma) of the 75 g oral glucose tolerance test is less than 140 mg/dl, is called IFG (impaired Fasting Glycemia).

The compound A can be used as an improving agent or an agent for the prophylaxis or treatment of diabetes, borderline type, impaired glucose tolerance, IFG (Impaired Fasting Glucose) and IFG (Impaired Fasting Glycemia) as defined by the above-mentioned new diagnostic criteria. Furthermore, compound A can be also used as a therapeutic agent for hypertension of hypertensive patients showing a level not less than the above-mentioned diagnostic criteria (e.g., fasting blood glucose level of 126 mg/dl). Moreover, compound A can be also used to prevent the progression of the borderline type, impaired glucose tolerance, IFG (Impaired Fasting Glucose) or IFG (Impaired Fasting Glycemia) to diabetes.

The insulin sensitizer etc. of the present invention can be used in combination with pharmaceutical agents such as a therapeutic agent for diabetes, a therapeutic agent for diabetic complications, an anti-hyperlipidemia agent, an antihypertensive agent, an anti-obesity agent, a diuretic, a chemotherapeutic agent, an immunotherapeutic agent, a therapeutic agent for osteoporosis, an anti-dementia agent, an erectile dysfunction amelioration agent, a therapeutic agent for urinary incontinence/urinary frequency and the like (hereinafter to be abbreviated as a combination drug). On such occasions, the timing of administration of the insulin sensitizer etc. of the present invention and that of the combination drug is not limited. They may be administered simultaneously or at staggered times to the administration subject. The dose of the combination drug can be appropriately determined based on the dose clinically employed. The mixing ratio of the compound of the present invention and the combination drug can be appropriately selected according to the administration subject, administration route, target disease, clinical condition, combination, and other factors. In cases where the administration subject is human, for example, the combination drug may be used in an amount of 0.01 to 100 parts by weight per part by weight of the compound A.

Examples of the therapeutic agent for diabetes include insulin preparations (e.g., animal insulin preparations extracted from the bovine or swine pancreas; human insulin preparations synthesized by a genetic engineering technique using E. coli or a yeast, and the like), other insulin sensitizers (e.g., pioglitazone hydrochloride, troglitazone, rosiglitazone, GI-262570, JTT-501, MCC-555, YM-440, KRP-297, CS-011, FK-614 etc.), α-glucosidase inhibitors (e.g., voglibose, acarbose, miglitol, emiglitate etc.), biguanides (e.g., phenformin, metformin, buformin etc.), insulin secretagogues [e.g., sulfonylureas (e.g., tolbutamide, glibenclamide, gliclazide, chlorpropamide, tolazamide, acetohexamide, glyclopyramide, glimepiride, glipizide, glybuzole etc.), repaglinide, senaglinide, nateglinide, mitiglinide or its calcium salt hydrate, GLP-1 etc.], amyrin agonists (e.g., pramlintide etc.), phosphotyrosine phosphatase inhibitors(e.g., vanadic acid etc.), dipeptidylpeptidase IV inhibitors (e.g., NVP-DPP-278, PT-100, P32/98 etc.), β3 agonists (e.g., CL-316243, SR-58611-A, UL-TG-307, SB-226552, AJ-9677, BMS-196085, AZ40140 etc.), gluconeogenesis inhibitors (e.g., glycogen phosphorylase inhibitor, glucose-6-phosphatase inhibitor, glucagon antagonist etc.), SGLT (sodium-glucose cotransporter) inhibitors (e.g., T-1095 etc.) and the like.

As the therapeutic agents for diabetic complications, for example, aldose reductase inhibitors (e.g., tolrestat, epalrestat, zenarestat, zopolrestat, minalrestat, fidarestat, SNK-860, CT-112 etc.), neurotrophic factors (e.g., NGF, NT-3, BDNF etc.), PKC inhibitors (e.g., LY-333531 etc.), AGE inhibitors (e.g., ALT946, pimagedine, pyratoxathine, N-phenacylthiazolium bromide (ALT766), EXO-226 etc.), active oxygen scavengers (e.g., thioctic acid etc.), cerebral vasodilators (e.g., tiapride, mexiletine etc.) and the like can be mentioned.

As the anti-hyperlipidemia agents, for example, statin compounds which are cholesterol synthesis inhibitors (e.g., cerivastatin, pravastatin, simvastatin, lovastatin, atorvastatin, fluvastatin, itavastatin or salts thereof (e.g., sodium salt etc.) etc.), squalene synthetase inhibitors or fibrate compounds having a triglyceride lowering effect (e.g., bezafibrate, clofibrate, simfibrate, clinofibrate etc.) and the like can be mentioned.

As the antihypertensive agents, for example, angiotensin converting enzyme inhibitors (e.g., captopril, enalapril, delapril etc.), angiotensin II antagonists (e.g., candesartan cilexetil, candesartan, losartan, losartan potassium, eprosartan, valsartan, termisartan, irbesartan, tasosartan, olmesartan, olmesartan medoxomil etc.), calcium antagonists (e.g., manidipine, nifedipine, amlodipine, efonidipine, nicardipine etc.), clonidine and the like can be mentioned.

As the anti-obesity agents, for example, central acting anti-obesity agent (e.g., dexfenfluramine, fenfluramine, phentermine, sibutramine, amfepramone, dexamphetamine, mazindol, phenylpropanolamine, clobenzorex etc.), pancreatic lipase inhibitors (e.g., orlistat etc.), β3 agonist (e.g., CL-316243, SR-58611-A, UL-TG-307, SB-226552, AJ-9677, BMS-196085, AZ40140 etc.), anorectic peptides (e.g., leptin, CNTF (ciliary neurotropic factor) etc.), cholecystokinin agonists (e.g., lintitript, FPL-15849 etc.) and the like can be mentioned.

As the diuretics, for example, xanthine derivatives (e.g., theobromine and sodium salicylate, theobromine and calcium salicylate etc.), thiazide preparations (e.g., ethiazide, cyclopenthiazide, trichlormethiazide, hydrochlorothiazide, hydroflumethiazide, benzylhydrochlorothiazide, penfluthiazide, poly 5 thiazide, methyclothiazide etc.), anti-aldosterone preparations (e.g., spironolactone, triamterene etc.), carbonic anhydrase inhibitors (e.g., acetazolamide etc.), chlorobenzenesulfonamide preparations (e.g., chlortalidone, mefruside, indapamide etc.), azosemide, isosorbide, ethacrynic acid, piretanide, bumetanide, furosemide and the like can be mentioned.

As the chemotherapeutic agents, for example, alkylation agents (e.g., cyclophosphamide, ifosphamide etc.), metabolic antagonists (e.g., methotrexate, 5-fluorouracil etc.), anticancer antibiotics (e.g., mitomycin, adriamycin etc.), plant-derived anticancer agents (e.g., vincristine, vindesine, taxol etc.), cisplatin, carboplatin, etoposide and the like can be mentioned. Of these, furtulon, neofurtulon etc., which are 5-fluorouracil derivatives, and the like are preferable.

As the immunotherapeutic agents, for example, microorganism or bacterial components (e.g., muramyl dipeptide derivative, picibanil etc.), polysaccharides having immunostimulant activity (e.g., lenthinan, schizophyllan, krestin etc.), cytokines obtained by genetic engineering techniques (e.g. , interferon, interleukin (IL) etc.), colony stimulating factor (e.g., granulocyte-colony stimulating factor, erythropoietin etc.) and the like can be mentioned, with preference given to IL-1, IL-2, IL-12 and the like.

As the therapeutic agents for osteoporosis, for example, alfacalcidol, calcitriol, elcaltonin, calcitonin salmon, estriol, ipriflavone, pamidronate disodium, alendronate sodium hydrate, incadronate disodium and the like can be mentioned.

As the anti-dementia agents, for example, tacrine, donepezil, rivastigmine, galantamine and the like can be mentioned.

As the erectile dysfunction amelioration agents, for example, apomorphine, sildenafil citrate and the like can be mentioned.

As the therapeutic agent for urinary incontinence/urinary frequency, for example, flavoxate hydrochloride, oxybutynin hydrochloride, propiverine hydrochloride and the like can be mentioned.

Moreover, pharmaceutical agents having a cachexia improving effect acknowledged in animal models and clinical situations, which include cyclooxygenase inhibitors (e.g., indomethacin etc.) [Cancer Research, Vol. 49, 5935-5939 pages, 1989], progesterone derivatives (e.g., megestrol acetate) [Journal of Clinical Oncology, Vol. 12, 213-225 pages, 1994], glucosteroid (e.g., dexamethasone etc.), metoclopramide pharmaceutical agents, tetrahydrocannabinol pharmaceutical agent (publications are the same as the above), fat metabolism improving agent (e.g., eicosapentanoic acid etc.) [British Journal of Cancer, Vol. 68, 314-318 pages, 1993], growth hormone, IGF-1, and antibodies against TNF-α, LIF, IL-6 and oncostatin M, which induce cachexia, and the like, can be also used in combination with the pharmaceutical agent of the present invention.

The combination drug preferably includes an insulin preparation, an insulin sensitizer, an α-glucosidase inhibitor, a biguanide agent, an insulin secretagogue (preferably sulfonylurea agent) and the like. Particularly, insulin sensitizers such as pioglitazone hydrochloride and the like are preferable.

The above-mentioned combination drug may be a combination of two ore more kinds thereof combined at appropriate ratios.

### Best Mode for Embodying the Invention

The present invention is explained in more detail in the following by referring to Examples and Experimental Examples, which are not to be construed as limitative.

The insulin sensitizer etc. of the present invention can be produced by, for example, the following prescriptions.

### (Examples)

### Example 1: capsule

| | | |
|---|---|---|
| (1) | Compound **A** | 30 mg |
| (2) | Lactose | 90 mg |
| (3) | Crystalline cellulose | 70 mg |
| (4) | Magnesium stearate | 10 mg |
| | 1 capsule | 200 mg |

(1), (2), (3) and 1/2 of (4) are admixed and granulated. Thereto is added the remaining (4), and the total amount is sealed in a gelatin capsule.

### Example 2 tablet

| | | |
|---|---|---|
| (1) | compound **A** | 30 mg |
| (2) | lactose | 35 mg |
| (3) | corn starch | 150 mg |
| (4) | microcrystalline cellulose | 30 mg |
| (5) | magnesium stearate | 5 mg |
| | 1 tablet | 250 mg |

(1), (2) , (3), 2/3 of (4) and 1/2 of (5) are admixed and granulated. Thereto are added the remaining (4) and (5), and the mixture is compression formed to give tablets.

### (Experimental Examples)

### Experimental Example 1: evaluation of insulin sensitivity in spontaneously hypertensive rats (SHR)

### (1) test method

For evaluation of insulin resistance, 23-week-old spontaneously hypertensive rats (SHR) were used. Vehicle (0.5% methyl cellulose solution administration) and compound A (0.3 and 1 mg/kg/day) were orally administered for 2 weeks, after which an insulin sensitivity evaluation test by a glucose clamp technique was performed. The rats were grouped such that each parameter was leveled based on the glucose level and plasma insulin value before start of the drug administration. SHR were fasted from the evening of the day before the operation of the glucose clamp technique.

The glucose clamp technique was performed according to the following procedures. The rats were anesthetized with pentobarbital sodium (50 mg/kg i.p.) and catheters [PR45 (trademark), NATUME SEISAKUSHO CO., LTD.] for blood collection, for intravenous injection of insulin [novolin R injection 40 (trademark), Novo Nordisk Pharma Ltd.] and for intravenous injection of glucose [Otsuka glucose injection 50% (trademark), Otsuka Pharmaceutical Co., Ltd.] were each placed in the right common carotid artery, left femoral vein and right femoral vein, respectively. The glucose level was measured using a simplified glucose meter [Advantage II (trademark), Roche Diagnostics Corporation]. Using a peristaltic pump [MINIPULS 3 (trademark), Gilson Company, Inc.], insulin was constantly infused continuously at an injection rate of 40 and 20 mU/kg/min for 5 and 3 min, respectively, to afford a high insulin state, and thereafter high insulin state was maintained at 8 mU/kg/min. Glucose was constantly infused intravenously to maintain the range of initial value±10% of blood glucose, using a different peristaltic pump. Glucose was continuously infused from 10 min after the start of insulin injection, and the glucose injection rate was changed after blood glucose measurement performed every 5 min. Glucose was constantly infused for 90 min and an average of glucose infusion rate for the period of 40 min from 50 min to 90 min after the start of the infusion was calculated and used as an index showing the insulin sensitivity (M-value). The higher the M-value is, the higher the insulin sensitivity is (insulin resistance is improved). For statistic analysis, Williams' test was used and significance was evaluated at a risk rate of less than 2.5%.

### (2) Results

M-value in the vehicle administration group (n=10), compound A 0.3 mg/kg administration group (n=10) and compound A 1 mg/kg administration group (n=10) was 5.9±0.5, 7.8±0.5 and 8.6±0.6 mg/kg/min, respectively. A significant increase was observed from the compound A 0.3 mg/kg administration group. From the above results, it has been clarified that compound A shows a dose-dependent insulin sensitivity enhancing effect in SHR.

### Industrial Applicability

Using the insulin sensitizer etc. of the present invention, insulin resistance, insulin resistance in hypertension and the like in mammals can be effectively improved. In addition, the insulin sensitizers etc. of the present invention are superior in safety and in the properties of a pharmaceutical product.

The 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof, which is an active ingredient in the present invention, has superior insulin resistance improving activity, and is useful as, for example, an insulin sensitizer, an impaired glucose tolerance improving agent, an agent for the prophylaxis or treatment of diabetes (e.g., type II diabetes etc.) or hyperinsulinemia, an agent for the prophylaxis or treatment of hypertension associated with insulin resistance, an agent for the prophylaxis or treatment of hypertension associated with impaired glucose tolerance, an agent for the prophylaxis or treatment of hypertension associated with diabetes, an agent for the prophylaxis or treatment of hypertension associated with hyperinsulinemia, an agent for improving insulin resistance occurring in association with hypertension, an agent for improving impaired glucose tolerance occurring in association with hypertension, an agent for the prophylaxis or treatment of diabetes occurring in association with hypertension, an agent for the prophylaxis or treatment of hyperinsulinemia occurring in association with hypertension, a therapeutic agent for hypertension of hypertension patient showing a fasting blood glucose level of not less than 126 mg/dl and the like.

This application is based on a patent application No. 369271/2001 filed in Japan, the contents of which are hereby incorporated by reference.

## Claims

1. An insulin sensitizer comprising a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

2. An impaired glucose tolerance improving agent comprising a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

3. An agent for the prophylaxis or treatment of diabetes, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

4. An agent for the prophylaxis or treatment of hyperinsulinemia, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

5. An agent for the prophylaxis or treatment of hypertension associated with insulin resistance, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

6. An agent for the prophylaxis or treatment of hypertension associated with impaired glucose tolerance, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

7. An agent for the prophylaxis or treatment of hypertension associated with diabetes, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

8. An agent for the prophylaxis or treatment of hypertension associated with hyperinsulinemia, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

9. An agent for improving insulin resistance occurring in association with hypertension, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

10. An agent for improving impaired glucose tolerance occurring in association with hypertension, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

11. An agent for the prophylaxis or treatment of diabetes occurring in association with hypertension, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

12. An agent for the prophylaxis or treatment of hyperinsulinemia occurring in association with hypertension, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

13. A therapeutic agent of hypertension, which is used for treating hypertension of a hypertensive patient showing a fasting blood glucose level of not less than 126 mg/dl, which comprises a 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof.

14. A method for treating hypertension, which comprises administering 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a hypertensive patient showing a fasting blood glucose level of not less than 126 mg/dl.

15. A method for improving insulin resistance, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

16. A method for improving impaired glucose tolerance, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

17. A method for the prophylaxis or treatment of diabetes, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

18. A method for the prophylaxis or treatment of hyperinsulinemia, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

19. A method for the prophylaxis or treatment of hypertension associated with insulin resistance, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

20. A method for the prophylaxis or treatment of hypertension associated with impaired glucose tolerance, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

21. A method for the prophylaxis or treatment of hypertension associated with diabetes, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

22. A method for the prophylaxis or treatment of hypertension associated with hyperinsulinemia, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

23. A method for improving insulin resistance occurring in association with hypertension, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

24. A method for improving impaired glucose tolerance occurring in association with hypertension, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

25. A method for the prophylaxis or treatment of diabetes occurring in association with hypertension, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

26. A method for the prophylaxis or treatment of hyperinsulinemia occurring in association with hypertension, which comprises administering an effective amount of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof to a mammal.

27. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an insulin sensitizer.

28. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an impaired glucose tolerance improving agent.

29. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of diabetes.

30. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hyperinsulinemia.

31. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hypertension associated with insulin resistance.

32. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hypertension associated with impaired glucose tolerance.

33. Use of 2-ethoxy-1-[[2'-(5-oxo-2,5-dihydro-2,2,4-oxadiazol-3-yl)biphenyl-4-yl]methyl]-1H-benzimidazole-7-carboxylic acid or a salt thereof for the production of an agent for the prophylaxis or treatment of hypertension associated with diabetes.

34. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hypertension associated with hyperinsulinemia.

35. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for improving insulin resistance occurring in association with hypertension.

36. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for improving impaired glucose tolerance occurring in association with hypertension.

37. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of diabetes occurring in association with hypertension.

38. Use of 2-ethoxy-1-[[2'-(5-oxo-2,5-dihydro-1,2,4-oxadiazol-3-yl)biphenyl-4-yl]methyl]-1H-benzimidazole-7-carboxylic acid or a salt thereof for the production of an agent for the prophylaxis or treatment of hyperinsulinemia occurring in association with hypertension.
